# EUROPÄISCHE PATENTSCHRIFT

(11) **EP 2 064 308 B1**
(45) Veröffentlichungstag und Bekanntmachung des Hinweises auf die Patenterteilung: **11.01.2012**
(21) Anmeldenummer: 06777124.6
(22) Anmeldetag: 30.08.2006
(51) Int. Cl.: C12M 1/107, C12M 1/02, B01F 7/00, B01F 15/00

(54) **RÜHRWERK FÜR EINEN FERMENTER, FERMENTER UND VERFAHREN ZUM BETREIBEN EINES FERMENTERS**
AGITATOR FOR A FERMENTER, FERMENTER AND METHOD FOR OPERATING A FERMENTER
MELANGEUR-AGITATEUR POUR FERMENTEUR, FERMENTEUR ET PROCEDE POUR ACTIONNER UN FERMENTEUR

(30) Priorität: 02.09.2005 DE 102005041798
(43) Veröffentlichungstag der Anmeldung: 03.06.2009
(73) Patentinhaber: Agraferm Technologies AG, 85276 Pfaffenhofen/Ilm (DE)
(72) Erfinder: FRIEDMANN, Johann, 85298 Scheyern/ Fernhag (DE); HECK, Christian, L-7794 Bissen (LU)
(74) Vertreter: Ganahl, Bernhard
(86) Internationale Anmeldenummer: PCT/EP2006/008488
(87) Internationale Veröffentlichungsnummer: WO 2007/025739

(56) Entgegenhaltungen:
- EP-A- 1 394 246
- DE-U1-202004 004 101
- DE-U1-202004 012 236

## Beschreibung

Die vorliegende Erfindung betrifft ein Rührwerk für einen Fermenter, einen Fermenter und einem Verfahren zum Betreiben eines Fermenters.

Zur Erzeugung von Biogas werden Fermenter als Gärbehälter verwendet, wobei das entstehende Biogas in Kesseln oder Motoren zur Stromerzeugung verbrannt wird. Als Ausgangsstoffe für die Biogaserzeugung kommen grundsätzlich alle Arten von Biomasse in Frage, deren Hauptkomponenten Kohlenhydrate, Eiweiße, Fette sowie Zellulose sind. Die für die Biogasproduktion nutzbaren organischen Stoffe sind zumeist Rest- oder Nebenprodukte verschiedener Branchen und Bereiche. Beispielsweise werden aus der Landwirtschaft Flüssig- und Festmist, Reststoffe der Pflanzenproduktion, aber auch extra hierfür angebaute Pflanzen wie beispielsweise Mais verwendet. Ferner können pflanzliche Rückstände der Brauerein und der gemüseverar beitenden Industrie sowie organische Schlämme und Abwasser der industriellen Aufarbeitung verwendet werden. Darüber hinaus ist es auch möglich, tierische Erzeugnisse oder Erzeugnisse der Kommunalentsorgung zu verwenden.

Biogas ist ein durch den anaeroben, mikrobioellen Abbau von organischen Stoffen entstehendes Gasgemisch, das zu 50 % bis 70 % aus dem hochwertigen Energieträger Methan (CH₄) besteht. Weitere Bestandteile sind 30 % bis 40 % Kohlenstoffdioxid (CO₂) sowie Spuren von Schwefelwasserstoff, Stickstoff, Wasserstoff und Kohlenstoffmonoxid.

Auf Grund des relativ hohen Energiegehalts lässt sich Biogas als Energieträger für die Wärme- und Krafterzeugung nutzen. Der durchschnittliche Heizwert von Biogas beträgt etwa 6 000 kcal/m³ (= 25 000 kJ/m³). Der Heizwert eines m³ Biogases entspricht demzufolge etwa 0,6 I Heizöl.

Bekannt ist es, in Biogasanlagen Fermenter einzusetzen, die beispielsweise ein Volumen von 150 m³ bis 3 000 m³ haben. Die Fermenter können im Einzellfall auch wesentlich größer sein. So ist ein Fermenter für eine Biogasanlage mit einem Volumen von 8 000 m³ bekannt. Im Fermenter verweilt das Substrat mehrere Tage, wobei durch die Aktivität der Mikroorganismen Biogas gebildet wird. Durch biochemische Umwandlungen wird im Fermenter das Biogas entschwefelt, wobei Schwefelwasserstoff in elementaren Schwefel umgewandelt wird wenn dem Fermenter im Gasraum Sauerstoff zugeführt wird. Um die Bildung von Schwimmdecken und Sinkschichten zu verhindern, wird das Substrat je nach Zusammensetzung gerührt. Hierdurch wird zusätzlich das Entweichen der entstehenden Gase erleichtert. Das vergorene Substrat wird anschließend in ein Endlager abgeführt, das möglichst geschlossen sein sollte, da Restbiogas ausgasen kann.

Das frisch erzeugte Biogas wird anschließend getrocknet und mit einem Sicherheitsfilter gereinigt. Die aus der Vergärung übrig gebliebene Biomasse eignet sich als biologischer Dünger. Bezüglich des Aufbaus einer Biogasanlage wird auf das Deutsche Gebrauchsmuster DE 20 2005 012 340 verwiesen.

Als Fermenter für die Trockenfermentation wurden bisher vor allem liegende Fermenter eingesetzt. Solche liegenden Fermenter sind beispielsweise aus einem sehr langen, schlanken Hohlkörper ausgebildet, in dem ein Rührwerk mit einer einzigen Welle angeordnet ist, das sich über die gesamte Länge des Fermenters erstreckt. Die Länge eines solchen Fermenters kann beispielsweise bis zu 25 m betragen. Die Enden der Rührwelle des Rührwerkes sind in den gegenüberliegenden Wandungen des Fermenterkörpers gelagert. Eine derart lange Rührwelle ist mechanisch schwer beherrschbar, da beträchtliche Momente an der Welle auftreten. Deshalb gibt es auch Fermenter, mit mehreren Rührwellen, die jeweils horizontal verlaufend und quer zur Längsrichtung des Fermenters angeordnet sind. Die mechanische Beanspruchung der einzelnen Rührwellen ist hierbei wesentlich geringer. Jedoch ist für jede Rührwelle ein separater Antriebsmechanismus vorzusehen, was wiederum erhebliche Kosten verursacht. Weiterhin ist die Abdichtung der Lagerung der Wellen in den Seitenwandungen des Fermenters höchst problematisch. Auch dieser Fermenter bildet einen langgestreckten Kanal, bei dem an einem Ende die Inputstoffe zugeführt werden und am anderen Ende die Gärprodukte abgezogen werden. Ein solcher Kanal weist üblicherweise einen rechteckförmigen Querschnitt auf und ist aus Betonsegmenten zusammengesetzt. Die Herstellung eines rechteckförmigen Betonkanals ist teuer.

Unabhängig von der Bauweise des Fermenters besteht bei diesen bekannten Fermentern das Problem, dass zum Austauschen eines Rührwerkes es notwendig ist, den Fermenter zu entleeren, um die sich im Fermenterkörper befindlichen Lagerstellen zugänglich zu machen. Da die Rührwerke einem unvermeidlichen Verschleißen unterliegen, muss der Betrieb des Fermenters in regelmäßigen Abständen unterbrochen werden um die Rührwerke warten zu können.

Aus der DE 20 2004 004 101 U1 geht ein Fermenter für eine Biogasanlage mit einer Rühreinrichtung hervor, der eine vertikal stehende Rührwelle aufweist, die an ihrem unteren Ende als Hohlwelle ausgebildet ist, so dass sie auf ein ortsfest im Fermenter angeordnetes Führungsrohr aufsteckbar ist. An der Rührwelle sind schwenkbar ausgebildete Rührpaddel angeordnet, so dass die Rührwelle einfach vertikal für Wartungs- oder Reparaturarbeiten entnommen werden kann.

Aus der DE 20 2004 012 236 U1 geht ein weiteres Rührwerk mit schräg angeordneter Drehwelle hervor. In dieser Dreh- bzw. Rührwelle ist am unteren Ende eine Lagereinheit integriert, so dass bei Service- oder Reparaturarbeiten das Lager zusammen mit der Drehwelle aus dem Fermenter entfernt wird.

Die DE 20 2004 005 331 U1 beschreibt ein Rührwerk, das über eine Öffnung in einer Seiten- oder Deckenwandung eines Behälters eines Fermenters herausgenommen werden kann. Dieses Rührwerk weist ein langgestrecktes Rohr auf, dessen Ende lose in ein Stecklager eingesetzt ist. In dem Rohr ist eine Welle gelagert, die an der Außenseite des Rohres angeordnete Rührelemente in Drehung versetzt.

In der DE 44 19 782 A1 ist ein horizontaler Fermenter beschrieben, der mit einer mittig abgestützten Rührwerkswelle versehen ist. Die Welle ist in der Mitte geteilt und deren jeweilige Enden werden durch ein Mittellager abgestützt.

Aus der DE 10 2004 027 077 A1 geht ein weiteres Rührwerk für einen Fermentationsbehälter hervor, das schräg in einen Behälter einsetzbar ist. Mit dem freien Ende ist das Rührwerk auf einem Montagebock abgestützt und gegenüber dem Montagebock schwenkbar angeordnet.

Die DE 20 2004 017 610 U1 beschreibt eine Biogasanlage mit einem Fermenter, in dem ein Rührwerk mit einer horizontal liegenden Welle vorgesehen ist. Bei der Ausführungsform wird an der Deckenwandung das zu fermentierende Gut zugeführt und im Bereich des Bodens ist eine Entnahmeöffnung zum Abziehen des fermentierten Gutes angeordnet.

In der DE 201 21 701 U1 ist eine Vorrichtung zum Abbau organischer Substanzen beschrieben, die einen Reaktor bzw. Fermenter aufweist, bei dem organische Substanzen schwimmend von einer Zuführöffnung bis zu einer entfernt davon angeordneten und im Wesentlichen auf dem selben horizontalen Niveau befindlichen Entnahmeöffnung transportiert werden. Hierdurch soll eine quasi-kontinuierliche Verfahrensführung ermöglicht werden. Auch kann eine Zerkleinerung der organischen Substanzen entfallen.

Die DE 201 11 480 U1 zeigt einen Fermenter mit einem Rührwerk, das um eine vertikal stehende Rührwelle gedreht wird. Ein Stück entfernt von der Welle sind Rührmittel angeordnet, die den Inhalt des Fermenters in Bewegung halten. Am Randbereich des Fermenters ist in dem Boden ein Sumpf eingelassen, in den die sich am Boden des Fermenters bildenden Sedimente zugeführt werden. Aus dem Sumpf werden die hier abgelagerten Sedimente mittels einer Rohrleitung abgezogen.

Aus der DE 200 11 783 U1 geht ein ähnlich ausgebildeter Fermenter hervor, der am Randbereich mit einer Art Sumpf versehen ist, aus der mittels einer Förderschnecke die im Fermenter abgelagerten Sedimente entfernt werden können.

Die DE 102 24 665 A1 zeigt einen Fermenter in Form eines geschlossenen Behälters, bei dem oben das zu fermentierende Material zugeführt wird und unten abgezogen wird. Dieser Fermenter ist mit um eine senkrechte Achse drehbaren Rührmitteln versehen.

Die DE 31 38 452 A1 zeigt einen weiteren Fermenter mit einem zylinderförmigen Fermenterbehälter, der am oberen Bereich des Behälters ein Einlassrohr und am unteren Bereich des Behälters diametral gegenüberliegend ein Auslassrohr aufweist.

Aus der EP 1 394 246 A1 geht eine Rühreinrichtung für einen Fermenter hervor, der einen zylinderförmigen Fermenterbehälter aufweist, wobei an jedem Fermenterbehälter radial außen zwei Rührwellen angeordnet sind, mit welchen das darin enthaltene Gut sowohl lokal verwirbelt als auch im gesamten Behälter umgewälzt wird.

Dieser Fermenter kann insbesondere mit einem Rührmodul versehen sein, in dem die beiden Rührwellen und der Antriebsmotor integriert sind. Das Rührmodul kann als Einheit für Reparatur- und Wartungsarbeiten aus dem Fermenter vertikal herausgezogen werden. Am Boden des Fermenters sind Aufnahmen angeordnet, um die Enden der Rührwellen aufzunehmen.

Die EP 0 307 500 A1 beschreibt einen transportablen Biogas-Erzeuger mit einem an einem Fahrzeug angeordneten, schräg gestellten, zylinderförmigen Behälter, der an einer Stirnwandung einen Einfüllstutzen aufweist und im Mantelbereich mit einer Entleeröffnung versehen ist.

Die DE 196 21 914 C1 zeigt einen weiteren Fermenter mit einem Art Sumpf in der Mitte des Fermenters, aus welchem mittels eines Schneckenantriebes Sinkschichten aus dem Fermenter entfernt werden können.
Der Erfindung liegt die Aufgabe zu Grunde, ein Rührwerk für einen Fermenter, einen Fermenter und ein Verfahren zum Betreiben eines Fermenters zu schaffen, die einen einfacheren und effizienteren Betrieb des Fermenters erlauben.

Die Aufgabe wird durch ein Rührwerk mit den Merkmalen des Anspruchs 1, einen Fermenter mit den Merkmalen des Anspruchs 8 und einem Verfahren mit den Merkmalen des Anspruchs 10 oder des Anspruchs 15 gelöst. Vorteilhafte Ausgestaltungen der Erfindung sind in den jeweiligen Unteransprüchen angegeben.

Das erfindungsgemäße Rührwerk für einen Fermenter, insbesondere einem Fermenter für die Trockenfermentation, umfasst
zumindest eine etwa vertikal angeordnete Rührwelle, an welcher zumindest ein Paddel angeordnet ist,
einen Antriebsmechanismus zum Drehen der Rührwelle, wobei der Antriebsmechanismus am oberen Endbereich der Rührwelle angreift, und
ein Zentrierlager zum Zentrieren des unteren Endes der Rührwelle, wobei das Zentrierlarger derart lösbar von der Rührwelle ausgebildet ist, dass diese durch Einscheiben in das Zentrierlager zentrierbar ist und alleine durch die Schwerkraft der Rührwelle im Zentrierlager gehalten wird, und das Zentrierlager einen Einführtrichter aufweist und mit einem Zentrierabschnitt versehen ist, wobei der Zentrierabschnitt unterhalb des Einführtrichters angeordnet ist, und am unteren Endbereich der Rührwelle ein Wellenstummel angeordnet ist, der drehbar gegenüber der übrigen Rührwelle mittels eines Lagers gelagert ist, und der Wellenstummel ein Koppelelement aufweist, das mit etwas Spiel formschlüssig in den Zentrierabschnitt eingreift.

Mit diesem Rührwerk ist es möglich, die Rührwelle während des Betriebes des Fermenters aus dem Zentrierlager nach oben herauszuziehen und aus dem Fermenter zu entfernen. Die Rührwelle kann dann gewartet und wieder in den Fermenter in das Zentrierlager eingeführt werden oder durch eine andere Rührwelle ausgetauscht werden. Hierbei muss der Fermenter nicht entleert werden, weshalb die Rührwelle wesentlich schneller austauschbar ist, als dies bei herkömmlichen Fermentern der Fall ist. Weist der Fermenter eine oder mehrere weitere Rührwellen auf, so kann der Betrieb weiter aufrecht erhalten werden.

Das Zentrierlager weist einen Einführtrichter auf, der das Einführen der Rührwelle in das Zentrierlager erleichtert. Der Einführtrichter ist ortsfest im Fermenter angeordnet, so dass er grundsätzlich eine beliebige Größe aufweisen kann. Es sind Einführhilfen an Rührwellen bekannt. Diese haben jedoch den Nachteil, dass sie bei einem mit Substrat gefüllten Fermenter das Substrat verdrängen müssen. Bei dem erfindungsgemäßen Rührwerk wird lediglich die relativ dünne lang gestreckte Rührwelle mit den Paddeln durch das Substrat hindurch bewegt.

Weiterhin ist am unteren Endbereich der Rührwelle ein Wellenstummel angeordnet, der mittels eines Lagers drehbar gegenüber der übrigen Rührwelle gelagert ist. Dieses Lager zum Drehen der Welle stellt das wartungsintensivste Teil der Rührwelle dar. Bei der Anordnung des Lagers in der Rührwelle kann das Lager zusammen mit der Rührwelle aus dem Fermenter herausgezogen und gewartet werden. Da der Wellenstummel mit einem Koppelelement versehen ist, das mit etwas Spiel formschlüssig in einen Zentrierabschnitt des Zentrierlagers eingreift, ist der Wellenstummel im Fermenter drehfest angeordnet, so dass die Rührwelle mittels des zwischen dem Wellenstummel und der übrigen Rührwelle vorgesehenen Lagers definiert gelagert ist.

Vorzugsweise weist das Zentrierlager im unteren Bereich eine Öffnung auf, aus welcher Material beim Einführen der Rührwelle in das Zentrierlager verdrängt werden kann.

Weiterhin können an der Rührwelle mehrere Rührpaddel angeordnet sein, die mit unterschiedlichen Neigungen gegenüber der Vertikalen einstellbar sind. So kann die Verdrängungswirkung der Viskosität des im Fermenter befindlichen Substrates angepasst werden. Hierbei ist es auch möglich mehrere Rührpaddel unterschiedlicher Neigung an der Rührwelle anzuordnen, so dass unterschiedlich stark viskose Substratschichten entsprechend unterschiedlich stark beaufschlagt werden.

Werden die Rührpaddel im wesentlichen vertikal eingestellt, so vermischen sie das Substrat lediglich in Radialrichtung. Hierdurch können somit einzelne Schichten im Fermenter individuell angesprochen werden. Werden die Rührpaddel gegenüber der Vertikalen geneigt, so wird das Substrat je nach Neigung zunehmend in Vertikalrichtung durchmischt. Die Einstellung der Neigung der Rührpaddel erlaubt somit eine Steuerung der Substratströme im Fermenter. Die Kombination aus vertikal angeordneter Welle und im Neigungswinkel einstellbarere Rührpaddel erlaubt ein gezieltes schichtweises Ansprechen des Substrates.

Der erfindungsgemäße Fermenter umfasst
ein Gehäuse mit zumindest einer Bodenplatte und einer oder mehreren die Bodenplatte umschließenden Seitenwandungen,
ein erfindungsgemäßes Rührwerk zum Rühren des im Fermenter befindlichen Substrats,
eine Eintragseinrichtung zum Zuführen von Inputstoffen, und
eine Entnahmeöffnung, wobei die Eintragseinrichtung zum Zuführen der Inputstoffe am oberen Bereich des Fermenters und die Entnahmeöffnung am unteren Bereich des Fermenters angeordnet sind.

Der erfindungsgemäße Fermenter wird somit von oben nach unten durchströmt, was einen kontinuierlichen Betrieb ermöglicht, wobei sich im Fermenter drei Zonen mit unterschiedlich dichtem Material bilden können. Die oberste Zone ist die Verflüssigungszone, in welcher das eingebrachte Substrat verflüssigt wird. Die mittlere Zone ist die Methanisierungszone, in welcher das bereits verflüssigte und etwas verdichtete Material den größten Anteil an Methan freisetzt. In der untersten Zone, der Entnahmezone, befindet sich das bereits vollständig bzw. fast vollständig abgebaute Substrat, das die größte Dichte aufweist. Durch die Durchströmung von oben nach unten wird eine hydraulische Entkopplung der Abbauschritte erzielt, wodurch die Biogasausbeute optimiert wird.

Bei der bevorzugten Ausführungsform des erfindungsgemäßen Fermenters ist das Rührwerk mit einer vertikal angeordneten Rührwelle ausgebildet. Hierdurch werden mit der Rührwelle verbundene Rührpaddel jeweils in einer horizontalen Ebene bewegt und das im Fermenter befindliche Substrat wird in Horizontalebenen gerührt. Dies fördert die Einstellung der oben beschriebenen geschichteten Abbauzonen.

Das erfindungsgemäße Verfahren zum Betreiben eines Fermenters, insbesondere eines Fermenters für die Trockenfermentation für Biogasanlagen, zeichnet sich dadurch aus, dass am oberen Bereich des Fermenters Inputstoffe für die Trockenfermentation zugeführt werden, und dass am unteren Bereich des Fermenters das vergorene Substrat abgezogen wird.

Durch die Durchströmung von oben nach unten wird die oben erwähnte Zonenbildung ermöglicht.

Vorzugsweise wird das Substrat des Fermenters radial vermischt, was die Zonenbildung fördert. Hierzu ist es auch förderlich, dass das Rühren langsam, beispielsweise mit einer Drehgeschwindigkeit der Rührwelle(n) im Bereich von 0 bis 20 U/min bzw. bis maximal 60 U/min durchgeführt wird.

Wird der Fermenter mit dieser Zonenbildung betrieben, so wird das oben zugeführte Edukt allmählich zum Produkt umgesetzt, wobei es gleichzeitig im Fermenter absinkt. Derartige Fermenter werden auch als Rohrreaktor bzw. als Pfropfstromreaktor bezeichnet. Die Einstellbarkeit der Neigung der Rührpaddel erlaubt jedoch auch einen anderen Betrieb der Fermenter, bei dem das Substrat vertikal von oben nach unten bzw. von unten nach oben durchmischt wird.

Vorzugsweise wird der Fermenter mit der oben erwähnten Zonenbildung betrieben, wobei jedoch im oberen Bereich eine gewisse Neigung der Rührpaddel eingestellt wird, so dass frisch zugeführtes Edukt intensiver mit dem bereits im Fermenter befindlichen Substrat vermischt wird.

Die Erfindung wird nachfolgend beispielhaft näher anhand der Zeichnungen erläutert. In den Zeichnungen zeigen schematisch:
- Figur 1: einen erfindungsgemäßen Fermenter in einer Schnittdarstellung zusammen mit einer Eintragseinrichtung,
- Figur 2: einen Schnitt durch den Fermenter aus Figur 1 im Bereich eines Rührwerks,
- Figur 3: im Teilschnitt den unteren Endbereich eines Rührwerks,

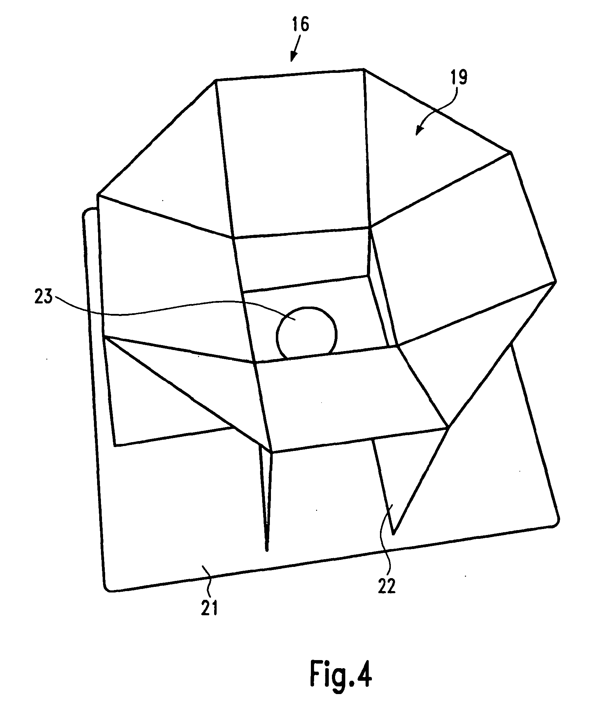

- Figur 4: das Zentrierlager mit einem Einführtrichter aus den Figuren 1 bis 3 mit Blickrichtung von schräg oben,
- Figur 5: das Einführen eines Rührwerks in den Fermenter in sechs Teilschritten,
- Figur 6: den Fermenterkörper mit teilweise geöffneter Decke,
- Figur 7: einen Schnitt durch den Fermenterkörper aus Figur 6 in einem unteren Eckbereich,

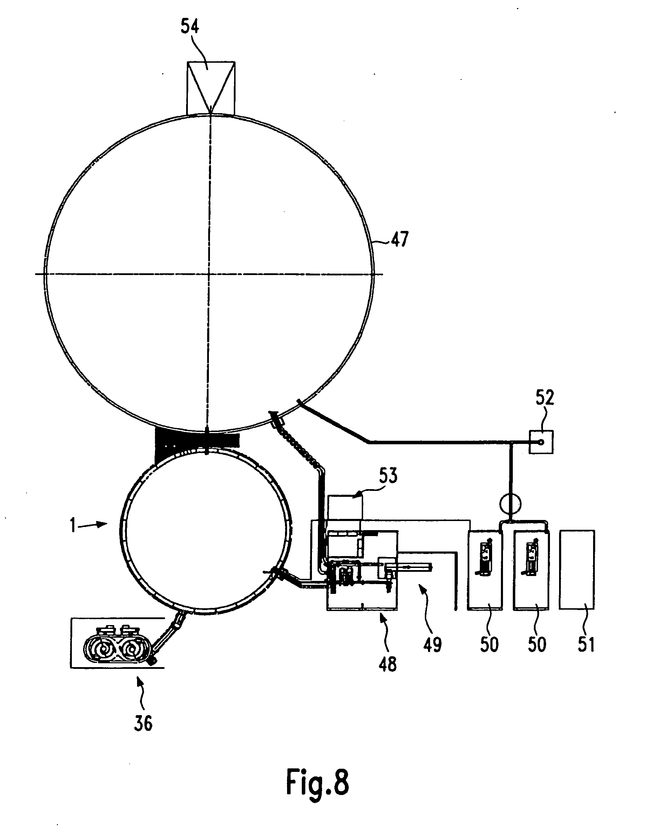

- Figur 8: den Aufbau einer Biogasanlage mit dem erfindungsgemäßen Fermenter,

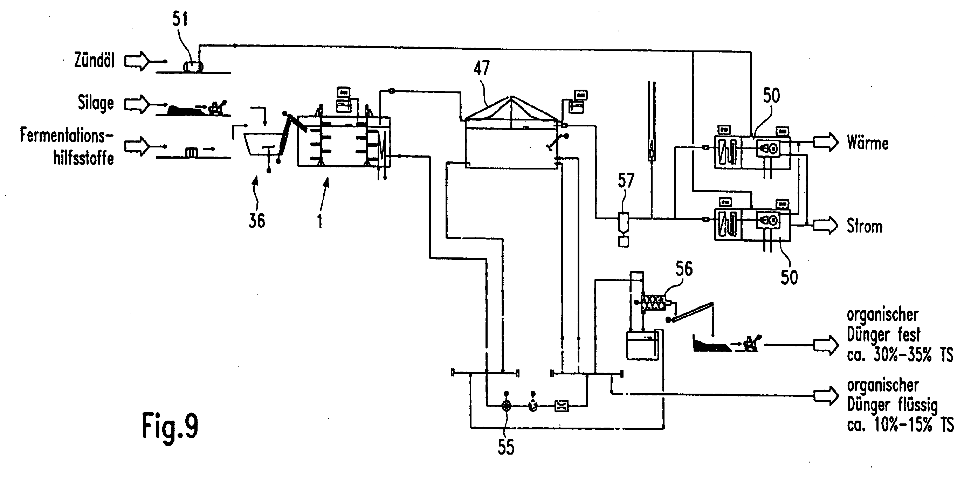

- Figur 9: ein Verfahrensfließbild der in der Biogasanlage aus Figur 8 ablaufenden Prozesse, und
- Figur 10: die Strömungen im Fermenter aus Figur 1.

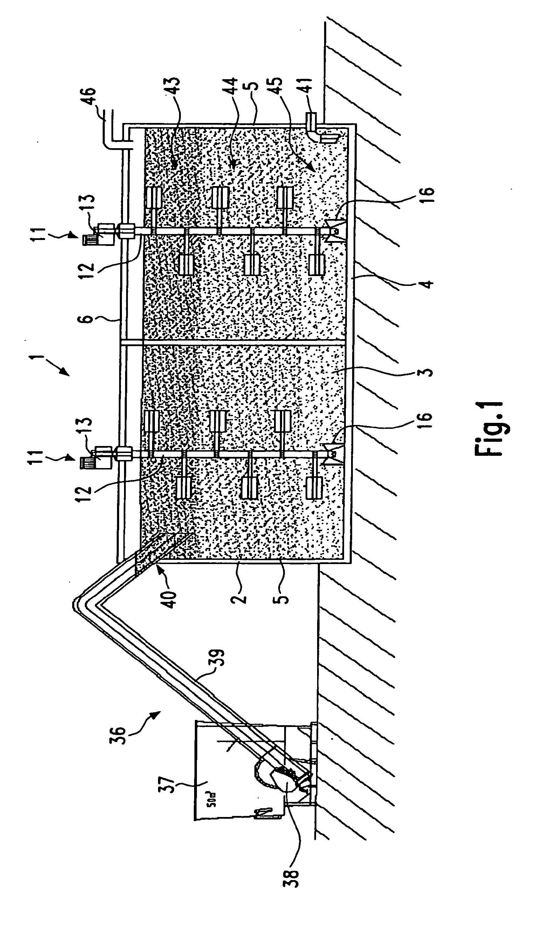

Ein Fermenter 1 weist eine Fermenterkörper 2 zur Aufnahme eines Substrates 3 auf (Figur 1).

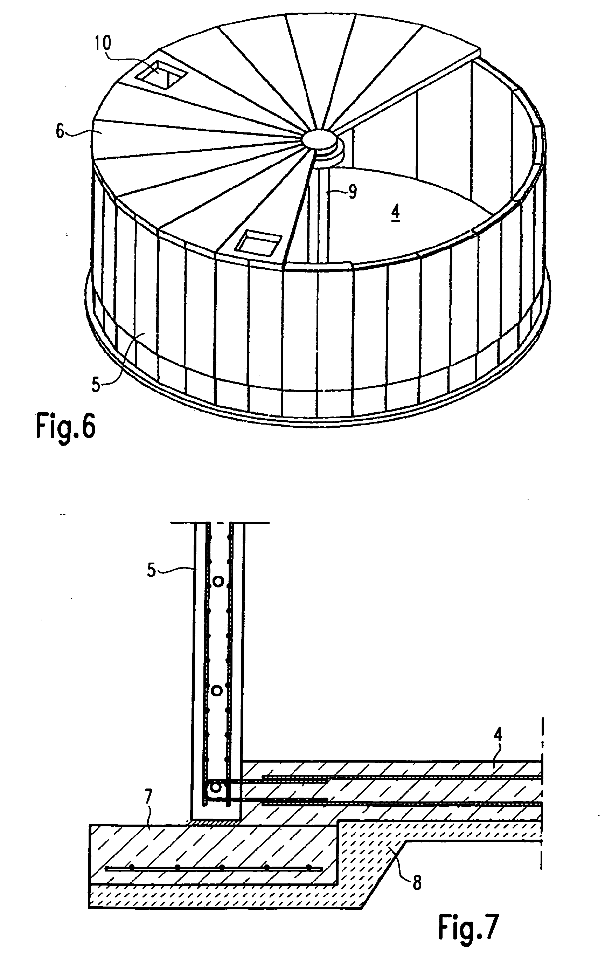

Der Fermenterkörper 2 ist aus einer in der Draufsicht kreisförmigen Bodenplatte 4, einer die Bodenplatte 4 umschließenden Seitenwandungen 5 und einer Decke 6 ausgebildet (Figur 6, 7). Die Seitenwandungen 5 und die Decke 6 sind aus Fertig-Betonteilen zusammengesetzt.

Figur 7 zeigt eine Schnittdarstellung durch einen unteren Eckbereich des Fermenter körpers mit einem Ringfundament 7, der aus Stahlbeton ausgebildeten Bodenplatte 4 und der aus Stahlbeton ausgebildeten Seitenwandungen 5. Das Ringfundament 7 und die Bodenplatte 4 lagern auf einer aus Makrobeton hergestellten Sauberkeitsschicht 8. Die einzelnen Beton-Fertigteile der Seitenwandungen 5 sind miteinander verspannt, wobei in den Segmenten der Seitenwandungen 5 Kanäle ausgebildet sind, in welchen die Spanngurte (nicht dargestellt) verlaufen.

Auch die Decke ist aus mehreren Beton-Fertigteilen hergestellt, die jeweils einzelne Kreissegmente bilden. Die Deckenteile lagern mit dem breiten Ende auf der Seitenwandung 5 und mit dem schmalen Ende auf einer zentrisch im Fermenterkörper 2 angeordneten Stützsäule 9. Einige Segmente der Decke 6 sind mit einer Öffnung 10 versehen, deren Funktion unten näher erläutert wird.

Der Fermenter 1 weist zumindest ein Rührwerk 11 auf. Das Rührwerk 11 umfasst eine etwa vertikal angeordnete Rührwelle 12,
einen Antriebsmechanismus 13, der am oberen Endbereich der Rührwelle 12 angreift,
mehrere Rührpaddel 14, die jeweils mittels einer Paddelstange 15 an der Rührwelle 12 befestigt sind, und
ein Zentrierlager 16, das auf der Bodenplatte 4 zur Aufnahme des unteren Endbereichs der Rührwelle 12 angeordnet ist.

Der Antriebsmechanismus 13 ist aus einem Elektromotor und einem Antriebsgetriebe ausgebildet und ist oberhalb der Decke 6 angeordnet. Der Antriebsmechanismus 13 ist an einer Deckelplatte 17 befestigt, mit der die Öffnung 10 der Decke 6 abgedeckt ist. Die Durchführung der Rührwelle 12 durch die Deckelplatte 17 erfolgt mit einem herkömmlichen Lager. Zusätzliche Abdichtungselemente sind nicht notwendig, da das Substrat im Normalbetrieb nicht mit der Decke 6 und der Deckelplatte 17 in Kontakt kommt.

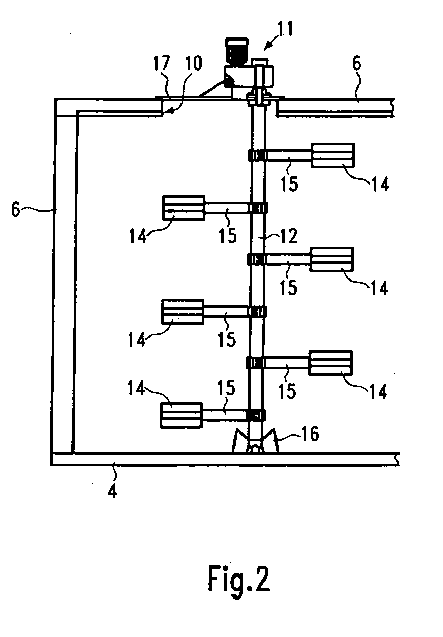

Die Rührwelle 12 ist aus einem Stahlrohr ausgebildet, das sich von der Decke 6 bis knapp über die Oberfläche der Bodenplatte 4 erstreckt. Die Paddelstangen 15 sind an die Rührwelle 12 geklemmt. Diese Klemmung kann an einer an sich beliebigen Stelle der Rührwelle 12 erfolgen. Deshalb ist es möglich, die Anzahl und die Anordnung der Rührpaddel zu variieren. Die Rührpaddel müssen nicht, wie es in Figur 1 und 2 gezeigt ist, in einer Ebene angeordnet sein. Sie können in beliebigen Winkel zueinander versetzt an der Rührwelle 12 angeordnet sein.

Die Rührpaddel 14 und die Paddelstangen 15 weisen jeweils Flansche mit korrespondierenden Bohrungen auf (nicht dargestellt), die mittels Schraubverbindung miteinander befestigbar sind. Hierdurch ist es auch möglich, die Rührpaddel 14 gegenüber der Vertikalen mit unterschiedlichen Neigungswinkeln an den Paddelstangen 15 zu befestigen. Hierdurch kann die effektive Verdrängungsfläche der Rührpaddel 14 in dem im Fermenter befindlichen Substrat 3 verändert werden. Je steiler die Rührpaddel 14 angeordnet sind, desto größer ist die effektive Verdrängungsfläche.

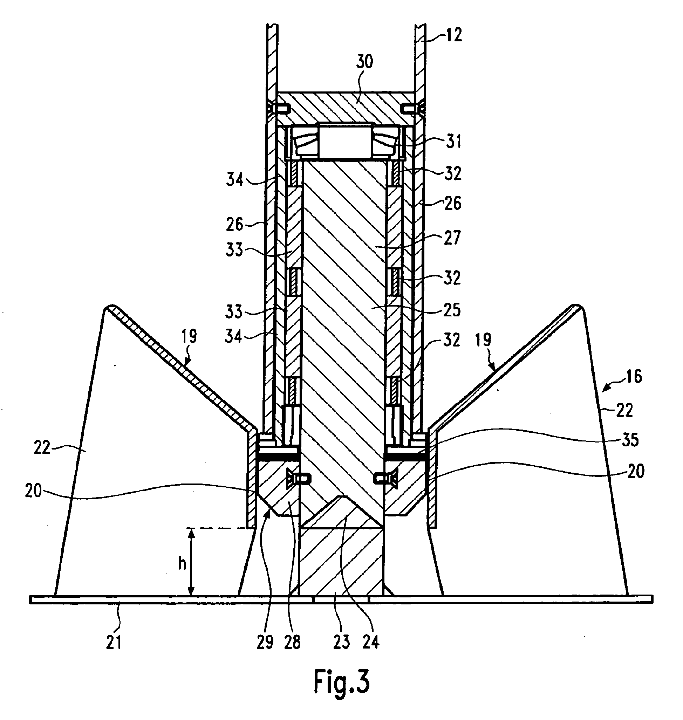

Figur 3 zeigt den sich im Zentrierlager 16 befindlichen unteren Endbereich der Rührwelle 12 in einer Schnittdarstellung. Das Zentrierlager 12 weist einen Einführtrichter 19 auf, der in den darunter angeordneten Zentrierabschnitt mündet. Der Zentrierabschnitt 20 ist in der Draufsicht quadratisch und wird durch vier Seitenwandungen ausgebildet. Grundsätzlich ist jede Form für den Zentrierabschnitt möglich, die zu einer drehfesten In-Eingriffnahme mit einem korrespondierend ausgebildeten Koppelelement 28 führt. Insbesondere kann die Form des Zentrierabschnittes und des korrespondierenden Koppelelements die Form eines beliebigen anderen Polyeders annehmen. Der Zentrierabschnitt 20 ist mit einem Abstand h über einer Basisplatte 21 angeordnet. Der Zentrierabschnitt 20 und der Einführtrichter 19 werden von Stützwandungen 22 gehalten, die sich vom Zentrierabschnitt 20 etwa radial nach au-βen erstrecken. Da der Zentrierabschnitt 20 mit dem Abstand h über der Basisplatte 21 angeordnet ist, ist zwischen dem Zentrierabschnitt 20 und der Basisplatte 21 ein Freiraum bzw. sind mehrere Öffnungen ausgebildet, durch welche sich im Zentrierlager 16 befindliches Substrat hindurch gedrängt werden kann, wenn eine Rührwelle 12 in das Zentrierlager 16 eingeführt wird.

Im Zentrum des Zentrierlagers 12 ist ein Auflager 23 in Form eines massiven Metallsockels angeordnet. Das Auflager ist mit einer nach oben gerichteten Zentrierspitze 24 versehen.

Sowohl das Auflager 23 als auch das Zentrierlager 16 sind mit der Basisplatte 21 verschweißt und werden als Baueinheit an vorbestimmten Stellen auf der Bodenplatte 4 des Fermenters 1 befestigt. Da das Zentrierlager 16 ortsfest im Fermenter, angeordnet ist, kann der Einführtrichter 19 grundsätzlich mit beliebiger Größe vorgesehen werden. Ein großer Einführtrichter erleichtert erheblich das Einsetzen des Rührwerkes in den Fermenter. Die Zentrierspitze 24 bewirkt eine sehr exakte Ausrichtung des unteren Endbereiches der Rührwelle 12 im Fermenter.
Das untere Ende der Rührwelle 12 umfasst einen Wellenstummel 25, der drehbar in dem rohrförmigen Wellenkörper 26 der Rührwelle 12 gelagert ist. Der Wellenstummel 25 ist aus einem massiven, langgestreckten Stab 27 und einem am unteren Endbereich des Stabes 27 angeordneten Koppelelement 28 ausgebildet. Das Koppelelement 28 steht radial am Stab 27 vor und ist mit seinen äußeren Begrenzungsflächen in der Draufsicht derart quadratisch geformt, das es mit geringem Spiel in den Zentrierabschnitt 20 passt. Am unteren umlaufenden Rand des Koppelelements 28 sind Einführschrägen 29 ausgebildet. Die untere Stirnfläche des Stabes 27 ist als kegelförmige Ausnehmung ausgeformt, die formschlüssig auf die Zentrierspitze 24 passt. Der Wellenstummel 25 ist somit durch das Zentrierlager 16 und das Auflager 23 zentriert gelagert und die formschlüssige In-Eingriffnahme zwischen dem Zentrierabschnitt 20 und dem Koppelelement 28 bewirkt eine drehfeste Anordnung des Wellenstummels 25.

In dem rohrförmigen Wellenkörper 26 ist ein kreisscheibenförmiger Anschlag 30 etwas oberhalb der oberen Stirnfläche des Wellenstummels 25 angeordnet. Zwischen dem Anschlag 30 und dieser Stirnfläche befindet sich ein Pendelrollenlager 31, über das die Last der Rührwelle 12 auf den Wellenstummel 25 übertragen wird und das ein Drehen des Wellenkörpers 26 bezüglich des Wellenstummels 25 erlaubt.

Im Bereich zwischen dem Pendelrollenlager 31 und dem Koppelelement 28 sind Nadellager 32 und Kunststoffhülsen 33 abwechselnd angeordnet, die den Wellenstummel 25 umschließen. An der Innenfläche des Wellenkörpers 26 ist im Bereich unter halb des Anschlages 30 ein Einschubrohr 34 angeordnet, das einerseits zum Einschieben des Wellenstummels 25 mit den Lagern 31, 32 in den unteren Endbereich des Wellenkörpers 26 dient und zum anderen den Wellenstummel mit den Lagern 31, 32 und den Kunststoffhülsen exakt im Wellenkörper 26 positioniert.

Im Bereich zwischen dem Koppelelement 28, dem Einschubrohr 34 und dem untersten Nadellager 32 ist eine Dichtungsanordnung 35 vorgesehen, welche mehrere Dichtungselemente umfasst, vorgesehen, die das Eindringen von Substrat in den Zwischenbereich zwischen dem Wellenstummel 25 und dem Wellenkörper 26 verhindert.

Die erfindungsgemäße Rührwelle 12 kann während des Betriebes des Fermenters nach oben herausgezogen werden, wobei das Koppelelement 28 aus dem Zentierlager 16 herausgleitet. Im Fermenter 1 verbleiben lediglich das Zentrierlager 16 und das Auflager 23. Dies sind starke Stahlteile, die keinen nennenswerten Verschleiß unterliegen und nicht regelmäßig gewartet werden müssen. Die wesentlich wartungsintensiveren Lager 31, 32 werden zusammen mit der Rührwelle 12 aus dem Fermenter entfernt und können außerhalb des Fermenters gewartet werden, ohne dass der Betreib des Fermenters unterbrochen werden muss.

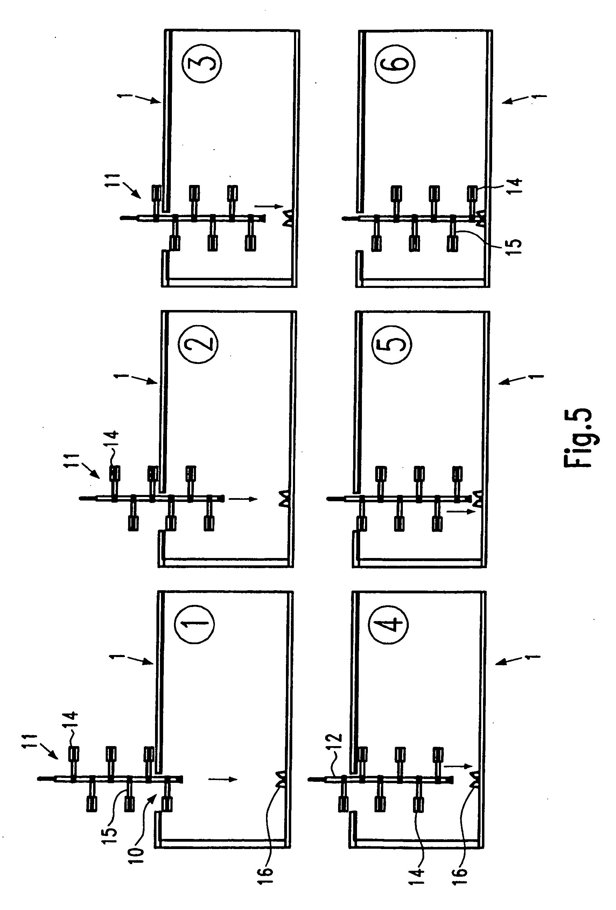

Beim Herausziehen und Einführen der Rührwelle sind die Rührpaddel 14 und die dazugehörigen Paddelstangen 15 jeweils so auszurichten, dass sie durch die Öffnung 10 der Decke 6 des Fermenters 1 geführt werden. Das Einführen des Rührwerkes 11 in den Fermenter 1 ist schematisch in Figur 5 dargestellt.

Im vorliegenden Ausführungsbeispiel weisen die Rührpaddel 14 und die Paddelstangen 15 eine gesamte Länge von jeweils 1,4 m auf. Die Öffnung 10 ist vorzugsweise mit einer geringfügig größeren Weite von z.B. 1,5 ausgebildet, wodurch die Festigkeit der Decke 6 nicht beeinträchtigt ist und zugleich das Rührwerk 11 mit der Rührwelle 12 herausgenommen und wieder eingeführt werden kann. Der vollständige Austausch eines Rührwerkes dauert wenige Stunden.

Zum Zuführen von Inputstoffen in den Fermenter 1 ist eine Eintragseinrichtung 36 vorgesehen (Fig. 1), die einen Vorratsbehälter 37, eine Förderschnecke 38 und einen Förderkanal 39 umfasst. Der Förderkanal mündet im oberen Bereich des Fermenters. Er kann entweder in einer Öffnung der Decke 6 münden, oder am oberen Randbereich der Seitenwandung 5. Im vorliegenden Ausführungsbeispiel ist die Öffnung 40 am oberen Randbereich der Seitenwandung 5 angeordnet. Zur Entnahme des vergorenen Materials aus dem Fermenter ist eine Entnahmeöffnung 41 am unteren Randbereich der Seitenwandung 5 des Fermenters 1 angeordnet. An der Entnahmeöffnung 41 ist eine Förderpumpe (nicht dargestellt) gekoppelt, die das vergorene Material zur weiteren Verarbeitung befördert.

Da die Zuführöffnung 40 oben und die Entnahmeöffnung 41 unten am Fermenter angeordnet sind, wird der Fermenter von oben nach unten beschickt. Vorzugsweise sind die Zuführöffnung 40 und die Entnahmeöffnung 41 diametral gegenüberliegend am Fermenter angeordnet, so dass das Substrat beim Durchfluss durch den Fermenter diesen einmal vollständig queren muss.

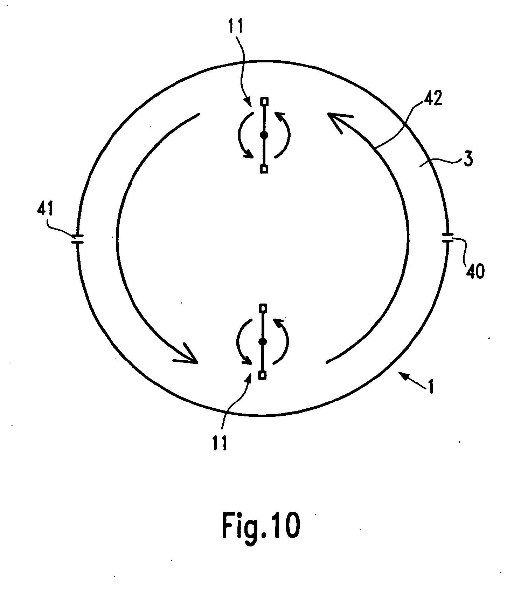

Der Betrieb des Fermenters 1 wird nachfolgend anhand der schematischen Darstellung aus Figur 10 näher erläutert, die eine Draufsicht des kreisförmigen Fermenter 1 mit zwei Rührwerken 11 zeigt.

Die Inputstoffe werden oben am Fermenter 1 durch die Zuführöffnung 40 zugeführt. Geeignete Inputstoffe für die Trockenfermentation sind im wesentlichen alle verwertbaren stapelfähigen Biomassen mit einem Trockenstoff-Gehalt von zumindest 25 %. Geeignete Inputstoffe sind z.B. Silomais, Getreide-GPS, Grassilage, Zuckerrübensilage, Futterrübensilage und Getreide (Rogen, Triticale, Gerste, Weizen).

Die beiden Rührwerke 11 werden in gleicher Drehrichtung betrieben. Die Drehgeschwindigkeit ist gering. Sie beträgt maximal 60 U/min. Typischerweise liegt die Drehgeschwindigkeit im Normalbetrieb im Bereich von 0 bis 20 U/min. Es hat sich gezeigt, dass bei dieser Anordnung mit vertikal ausgerichteter Rührwelle und einer langsamen, kontinuierlichen Drehung des Rührwerkes das gesamte Substrat 3 des Fermenters 1 bewegt wird (siehe Pfeile 42). Hierfür ist es vorteilhaft, wenn der Fermenter in seiner Draufsicht kreisförmigen ausgebildet ist.

Es hat sich zudem gezeigt, dass zum Bewegen des vollständigen Substrates lediglich ein einziges Rührwerk notwendig ist. Aus Sicherheitsgründen sind vorzugsweise jedoch zwei oder mehrere Rührwerke eingebaut, um beim Ausfall eines Rührwerkes das Substrat kontinuierlich in Bewegung halten zu können. Hierdurch wird ein Ansteigen des Flüssigkeitsspiegels im Fermenter durch Biogaseinschlüsse in den Schwimmdecken verhindert.

Bei der kontinuierlichen rotatorischen Umwälzung des Substrates im Fermenter stellen sich drei Abbauzonen 43, 44, 45 ein (Figur 1). Die drei Abbauszonen sind übereinander geschichtet. Die oberste Zone ist eine Verflüssigungszone 43, die mittlere Zone eine Methanisierungszone 44 und die untere Zone eine Entnahmezone 45.

Diese drei Abbauzonen stellen sich ein, wenn die Rührpaddel 14 im wesentlichen vertikal ausgerichtet sind, so dass im Substrat keine nennenswerte Bewegung nach oben oder unten erzeugt wird. Derart vertikal ausgerichtete Rührpaddel 14 sprechen das Substrat im wesentlichen nur in einzelnen Ebenen an, so dass die Ebenen nicht durchmischt werden. Vorzugsweise werden die obersten Paddel bzw. die sich in der Verflüssigungszone 43 befindlichen Rührpaddel 14 gegenüber der Vertikalen etwas schräg gestellt, so dass neu hinzugefügtes Edukt unverzüglich mit dem Substrat der Verflüssigungszone vermischt wird.

In einer anderen Betriebsart werden die Rührpaddel gegenüber der Vertikalen geneigt. Bei einem Neigungswinkel von 20° bis 70° und insbesondere von 30° bis 60° wird das Substrat im erheblichen Umfang vertikal durchmischt. Sind alle Rührpaddel einer Rührwelle in die gleiche Richtung geneigt, so entsteht eine vertikale Strömung des Substrates entlang der Rührwelle über die gesamte Füllhöhe des Substrates. Je nach der Richtung der Rührwelle ist die Strömung entlang der Rührwelle nach oben oder nach unten gerichtet. In einem solchen Betrieb stellen sich keine horizontalen Abbauzonen ein, sondern das gesamte Substrat wird gleichmäßig vermischt.

In der Verflüssigungszone befindet sich das noch wenig abgebaute Substrat, das aufgrund des hohen Organikgehalts die geringste Dichte aufweist. Das Material sinkt mit fortschreitender Verflüssigung durch den biologischen Abbau aus der Verflüssigungszone in die Methanisierungszone ab. In der Methanisierungszone wird das meiste Methan freigesetzt. Stark abgebautes Substrat gelangt auf Grund der höheren Dichte in die Entnahmezone, aus welcher es durch die Entnahmeöffnung 41 abgeführt wird.

Das hierbei entstandene Biogas sammelt sich unterhalb der Decke 6 an und wird an einer Öffnung 46 in der Decke 6 abgeführt.

Der Füllstand im Fermenter wird mittels einer Radarsonde (nicht dargestellt) überwacht. Übersteigt der Füllpegel ein bestimmtes Niveau, schaltet sich automatisch die Förderpumpe für die Entnahme des abgebauten Materials ein.

Der Fermenter 1 wird kontinuierlich mittels der Eintragseinrichtung 36 beschickt. Der Vorratsbehälter 37 der Eintragseinrichtung 36 kann vom Betreiber diskontinuierlich gefüllt werden, wobei die Steuereinrichtung die kontinuierliche Beschickung mittels der Förderschnecke 38 steuert. Mittels der durch die Radarsonde gesteuerten Entnahme erfolgt die Abförderung des abgebauten Materials auch kontinuierlich.

Die typische mittlere hydraulische Verweilzeit des Substrates beträgt etwa 40 Tage im Fermenter. Die spezifische Raumbelastung liegt bei 8 kg oTS/m³/d.

Vorzugsweise ist der Fermenter 1 mit einer Heizeinrichtung ausgestattet, die die Temperierung des Substrats im Fermenter erlaubt, so dass eine optimale Gärtemperatur im Fermenter gehalten werden kann.

Nachfolgend wird eine Biogasanlage mit dem erfindungsgemäßen Fermenter erläutert. Die Biogasanlage weist einen Fermenter 1 mit der Eintragseinrichtung 36, eine kombinierten Nachgärer/Gasspeicher 47, eine Pumpenstation 48, einen Lagerplatz für die festen Gärprodukte 49, zwei Blockheizkraftwerke 50, einen Öltank 51, eine Biogasnotfackel 52, eine Trafostation 53 und einen Abfüllplatz 54 für die flüssigen Gärprodukte.

Im Vorratsbehälter 37 der Eintragseinrichtung 36 wird Silage mit Fermentationshilfsstoffen gemischt und diese Mischung wird als Inputstoffe dem Fermenter 1 zugeführt. Das im Fermenter erzeugt Biogas wird über die Pumpenstation 48 dem kombinierten Nachgärer/Gasspeicher 47 zugeführt. Die vergärten Substrate werden über die Pumpenstation 48 entweder dem Nachgärer/Gasspeicher 47 direkt oder über ein Zerkleinerungsaggregat 55 dem Nachgärer/Gasspeicher 47 zugeführt. Der Nachgärer/Gasspeicher 47 ist ein großvolumiger Speicherbehälter mit einer Doppelmembran, wobei sich zwischen den beiden Membranen das Biogas befindet und unterhalb der unteren Membran das flüssige Gärprodukt. Das flüssige Gärprodukt kann dem Nachgärer/Gassspeicher 47 und/oder dem Fermenter 1 entnommen und über einen Separator 56 von seinen Festbestandteilen getrennt werden, die als organischer Dünger verwendbar sind, wonach das aufgetrennte flüssige Gärprodukt dem Nachgärer/Gasspeicher 47 zugeführt wird. Somit kann der Trockensubstanzgehalt im Nachgärer/Gasspeicher 47 durch den Seperator 56 gesteuert werden Dieser Dünger weist typischerweise einen Trockenstoffgehalt von 30 % bis 35 % auf.

Das im Fermenter 1 abgebaute Substrat kann auch direkt als organischer Dünger verwendet werden. Dies ist ein flüssiger Dünger mit einem Trockenstoffgehalt von 10 % bis 15 %.

Das im Nachgärer/Gasspeicher 47 gespeicherte Biogas wird über eine Kondensatabscheider 57 den Blockheizkraftwerken 50 zur Erzeugung von Strom und Wärme zugeführt. Der Öltank 51 ist dazu vorgesehen, die Blockheizkraftwerke 50 mit Zündöl zu versorgen. Weiterhin kann mit einer Notfackel 52 Biogas verbrannt werden, wenn die Blockheizkraftwerke die anfallende Biogasmenge nicht verarbeiten können.

Die Erfindung kann folgendermaßen kurz zusammengefasst werden:

Die Erfindung betrifft ein Rührwerk für einen Fermenter, einen Fermenter und ein Verfahren zum Betreiben eines Fermenters.

Das Rührwerk weist eine Rührwelle auf, die erfindungsgemäß etwa senkrecht im Fermenter steht. Hierdurch wird das im Fermenter befindliche Substrat in horizontalen Ebenen umgewälzt. Dies erlaubt die Einstellung mehrerer geschichteter Abbauzonen.

Weiterhin ist das Rührwerk vorzugsweise so ausgebildet, dass es während des laufenden Betriebes aus dem Fermenter nach oben herausgezogen werden kann. Hierdurch ist es nicht notwendig, für Wartungsarbeiten am Rührwerk den Fermenter zu entleeren.

### Bezugszeichenliste

- 1: Fermenter
- 2: Fermenterkörper
- 3: Substrat
- 4: Bodenplatte
- 5: Seitenwandung
- 6: Decke
- 7: Ringfundament
- 8: Sauberkeitsschicht
- 9: Stützsäule
- 10: Öffnung
- 11: Rührwerk
- 12: Rührwelle
- 93: Antriebsmechanismus
- 14: Rührpaddel
- 15: Paddelstange
- 16: Zentrierlager
- 17: Deckelplatte
- 18:
- 19: Einführtrichter
- 20: Zentrierabschnitt
- 21: Basisplatte
- 22: Stützwandungen
- 23: Auflager
- 24: Zentrierspitze
- 25: Wellenstummel
- 26: Wellenkörper
- 27: Stab
- 28: Koppelelement
- 29: Einführschräge
- 30: Anschlag

- 31: Pendelrollenlager
- 32: Nadellager
- 33: Kunststoffhülse
- 34: Einschubrohr
- 35: Dichtungsanordnung
- 36: Eintragseinrichtung
- 37: Vorratsbehälter
- 38: Förderschnecke
- 39: Förderkanal
- 40: Zuführöffnung
- 41: Entnahmeöffnung
- 42: Pfeil
- 43: Verflüssigungszone
- 44: Methanisierungszone
- 45: Entnahmezone
- 46: Öffnung
- 47: Nachgärer/Gasspeicher
- 48: Pumpenstation
- 49: Lagerplatz
- 50: Blockheizkraftwerk
- 51: Öltank
- 52: Biogasnotfackel
- 53: Trafostation
- 54: Abfüllplatz
- 55: Zerkleinerungsaggregat
- 56: Separator
- 57: Kondensatabscheider

## Patentansprüche

1. Rührwerk für einen Fermenter, mit
- zumindest einer etwa vertikal angeordneten Rührwelle (12), an welcher zumindest ein Rührpaddel (14) angeordnet ist,
- einem Antriebsmechanismus (13) zum Drehen der Rührwelle (12), wobei der Antriebsmechanismus (13) am oberen Endbereich der Rührwelle (12) angreift, und
- ein Zentrierlager (16) zum Zentrieren des unteren Endes der Rührwelle (12), und das Zentrierlager (16) derart lösbar von der Rührwelle (12) ausgebildet ist, dass diese durch Einschieben in das Zentrierlager (16) zentrierbar ist und alleine durch die Schwerkraft der Rührwelle im Zentrierlager (16) gehalten wird, wobei das Zentrierlager (16) einen Einführtrichter (19) aufweist und mit einem Zentrierabschnitt (20) versehen ist, wobei der Zentrierabschnitt unterhalb des Einführtrichters (19) angeordnet ist, und
am unteren Endbereich der Rührwelle (12) ein Wellenstummel (25) angeordnet ist, der drehbar gegenüber der übrigen Rührwelle mittels eines Lagers (31) gelagert ist, und der Wellenstummel (25) ein Koppelelement (28) aufweist, das mit etwas Spiel formschlüssig in den Zentrierabschnitt eingreift.

2. Rührwerk nach Anspruch 1,
**dadurch gekennzeichnet,**
**dass** die Rührpaddel (14) mit unterschiedlichen Neigungen gegenüber der Vertikalen einstellbar sind.

3. Rührwerk nach Anspruch 1 oder 2,
**dadurch gekennzeichnet,**
**dass** das Zentrierlager (16) einen Zentrierabschnitt (20) aufweist, der in der Draufsicht die Form eines Polyeders, insbesondere eines Quadrates, besitzt.

4. Rührwerk nach einem der Ansprüche 1 bis 3,
**dadurch gekennzeichnet,**
**dass** das Zentrierlager (16) am unteren Bereich eine Öffnung aufweist, aus welcher Material beim Einführen der Rührwelle (12) in das Zentrierlager (16) verdrängt werden kann.

5. Rührwerk nach einem der Ansprüche 1 bis 4
**dadurch gekennzeichnet,**
**dass** innerhalb des Zentrierlagers (16) ein Auflager (23) angeordnet ist, wobei das Auflager (23) mit einer nach oben gerichteten Zentrierspitze (24) versehen ist.

6. Rührwerk nach einem der Ansprüche 1 bis 5,
**dadurch gekennzeichnet,**
**dass** die Rührwelle (12) einen rohrförmigen Wellenkörper (26) umfasst, in dem der Wellenstummel (25) gelagert ist, wobei der Wellenstummel (25) ein Stück am Wellenkörper (26) vorsteht, und zwischen dem Wellenstummel (25) und dem Wellenkörper (26) ein oder mehrere Kugel-, Wälz-, Rollen- und/oder Nadellager (32) vorgesehen sind.

7. Rührwerk nach einem der Ansprüche 1 bis 6,
**dadurch gekennzeichnet,**
**dass** die Rührpaddel (14) an frei wählbaren Positionen der Rührwelle (12) an dieser befestigbar sind.

8. Fermenter, mit
- einem Gehäuse (2) umfassend zumindest eine Bodenplatte (4) und eine oder mehrere die Bodenplatte (4) umschließenden Seitenwandungen (5),
- einem Rührwerk (11), das gemäß einem der Ansprüche 1 bis 7 ausgebildet ist,
- einer Eintragseinrichtung (26) zum Zuführen von Inputstoffen, und
- eine Entnahmeöffnung (41 ),
wobei die Eintragseinrichtung (36) zum Zuführen der Inputstoffe am oberen Bereich des Fermenters (1) und die Entnahmeöffnung (41) am unteren Bereich des Fermenters (1) angeordnet sind.

9. Fermenter nach Anspruch 8,
**dadurch gekennzeichnet,**
**dass** die Entnahmeöffnung (41) am Fermenter (1) diametral gegenüberliegend zur Eintragseinrichtung (36) angeordnet ist, und
**dass** der Fermenter (1) mehrere Rührwerke (11) aufweist.

10. Verfahren zum Betreiben eines Fermenters, wobei
ein Fermenter nach Anspruch 8 oder 9 verwendet wird,
am oberen Bereich des Fermenters (1) Inputstoffe für eine Trockenfermentation zugeführt werden, und am unteren Bereich des Fermenters (1) das vergorene Substrat abgezogen wird.

11. Verfahren nach Anspruch 10,
**dadurch gekennzeichnet,**
**dass** die Inputstoffe einen Trockenstoff-Gehalt von mindestens 25% aufweisen.

12. Verfahren nach einem der Ansprüche 10 bis 11,
**dadurch gekennzeichnet,**
**dass** das Substrat des Fermenters (1) mit dem Rührwerk (11) radial vermischt wird, wobei sich mehrere übereinander geschichtete Abbauzonen (43, 44, 45) einstellen.

13. Verfahren nach einem der Ansprüche 10 bis 12,
**dadurch gekennzeichnet,**
**dass** die Neigung der Rührpaddel (14) gegenüber der Vertikalen im Bereich von 20° bis 70° und vorzugsweise im Bereich von 30° bis 60° liegt.

14. Verfahren nach einem der Ansprüche 10 bis 13,
**dadurch gekennzeichnet,**
**dass** die im Fermenter befindliche Rührwelle (12) mit einer Drehgeschwindigkeit von maximal 60 U/min betrieben wird.

15. Verfahren zum Betreiben eines Fermenters, wobei der Fermenter ein Gehäuse (2) mit einer Decke (6) und einem Rührwerk (11) nach einem der Ansprüche 1 bis 7 mit einer vertikalen Rührwelle und mehreren an der Rührwelle horizontal abstehenden Rührpaddel (14) aufweist, und in der Decke (6) eine Öffnung (10) ausgebildet ist, wobei
beim Einführen und beim Herausziehen des Rührwerkes (11) durch Drehen der Rührwelle die Rührpaddeln (14) einzeln bzgl. der Öffnung (10) ausgerichtet werden, so dass das Rührwerk durch eine Öffnung (10) eingeführt und herausgezogen werden kann, die geringfügig größer als die Länge der einzelnen Rührpaddel (14) ist.

## Claims

1. Agitator for a fermenter, with
- at least one vertical agitator shaft (12), to which is fitted one or more agitator paddles (14),
- a drive mechanism (13)to rotate the agitator shaft (12), with the drive mechanism (13) acting on the upper end section of the agitator shaft (12), and
- a centring bearing (16) for centring the lower end of the agitator shaft (12), wherein the centring bearing (16) is designed to be releasable from the agitator shaft (12) in such a way that the latter may be centred by pushing into the centring bearing (16) and held in the centring bearing (16) solely by the force of gravity of the agitator shaft, wherein the centring bearing (16) has an insertion funnel (19) and is provided with a centring section (20), wherein the centring section is below the insertion funnel (19) and
at the lower end section of the agitator shaft (12) there is provided a stub shaft (25), mounted by means of a bearing (31) so as to be rotatable relative to the rest of the agitator shaft, and the stub shaft (25) has a coupling element (28) which engages positively with some play in the centring section.

2. Agitator according to claim 1,
**characterised in that**
the agitator paddles (14) may be set at different angles relative to the vertical.

3. Agitator according to claim 1 or 2
**characterised in that**
the centring bearing (16) has a centring section (20) which, viewed from above, has the shape of a polyhedron, in particular a square.

4. Agitator according to any of claims 1 to 3,
**characterised in that**
the centring bearing (16) has in its lower part an opening through which material may be displaced on insertion of the agitator shaft (12) into the centring bearing (16).

5. Agitator according to any of claims 1 to 4,
**characterised in that**
there is a pedestal (23) within the centring bearing (16), wherein
the pedestal (23) is provided with a centring point (24) directed upwards.

6. Agitator according to any of claims 1 to 5,
**characterised in that**
the agitator shaft (12) comprises a tubular shaft body (26) in which the stub shaft (25) is mounted, with the stub shaft (25) projecting a short distance from the shaft body (26)
and one or more ball, roller and/or needle bearings (32) are provided between the stub shaft (25) and the shaft body (26).

7. Agitator according to any of claims 1 to 6,
**characterised in that**
the agitator paddles (14) may be attached to the agitator shaft (12) at positions which may be freely selected.

8. Fermenter, with
- a housing (2) comprising at least one base plate (4) and one or more side walls (5) surrounding the base plate (4),
- an agitator (11) designed in accordance with any of claims 1 to 7,
- a feeding device (26) for feeding n input materials, and
- a discharge outlet (41),
wherein the feeding device (36) for the feeding in of input materials is provided in the upper section of the fermenter (1), and the discharge outlet (41) is provided in the lower section of the fermenter (1).

9. Fermenter according to claim 8,
**characterised in that**
the discharge outlet (41) of the fermenter (1) is arranged diametrically opposite the feeding device (36), and
the fermenter (1) has several agitators (11).

10. Method of operating a fermenter, wherein
a fermenter according to claim 8 or 9 is used,
input materials for dry fermentation are fed into the upper part of the fermenter (1), and the fermented substrate is removed from the lower part of the fermenter (1).

11. Method according to claim 10,
**characterised in that**
the input materials have a dry substance content of at least 25%.

12. Method according to any of claims 10 to 11,
**characterised in that**
the substrate of the fermenter (1) is mixed radially by the agitator (11), resulting in the creation of several decomposition zones (43, 44, 45) in layers one above the other.

13. Method according to any of claims 10 to 12.
**characterised in that**
the inclination of the agitator paddles (14) from the vertical lies in the range between 20° and 70°, and preferably in the range between 30° and 60°.

14. Method according to any of claims 10 to 13,
**characterised in that**
the agitator shaft (12) in the fermenter is operated at a maximum speed of rotation of 60 rpm.

15. Method of operating a fermenter, , wherein the fermenter has a housing (2) with a roof (6) and an agitator (11) according to any of claims 1 to 7 with a vertical agitator shaft and several agitator paddles (14) extending horizontally from the agitator shaft, and with an opening (10) provided in the roof (6), wherein on insertion and removal of the agitator (11) by rotation of the agitator shaft the agitator paddles (14) are individually aligned relative to the opening (10) so that the agitator shaft may be inserted and removed through an opening (10) which is slightly larger than the length of the individual agitator paddles (14).

## Revendications

1. Agitateur pour un fermenteur, comprenant
- au moins un arbre d'agitateur (12) agencé approximativement verticalement et sur lequel est agencée au moins une pale d'agitateur (14),
- un mécanisme d'entraînement (13) pour faire tourner l'arbre d'agitateur (12), ledit mécanisme d'entraînement (13) attaquant la zone terminale supérieure de l'arbre d'agitateur (12), et
- un palier de centrage (16) pour centrer l'extrémité inférieure de l'arbre d'agitateur (12), ledit palier de centrage (16) étant réalisé de façon détachable vis-à-vis de l'arbre d'agitateur (12) de telle manière que celui-ci peut être centré en l'enfilant dans le palier de centrage (16) et est maintenu dans le palier de centrage (16) uniquement par la force de gravité sur l'arbre d'agitateur, le palier de centrage (16) comportant un entonnoir d'introduction (19) et étant pourvu d'un tronçon de centrage (20), ledit tronçon de centrage étant agencé au-dessous de l'entonnoir d'introduction (19), et
un moignon d'arbre (25) est agencé au niveau de la zone terminale inférieure de l'arbre d'agitateur (12), ce moignon étant monté en rotation par rapport au reste de l'arbre d'agitateur au moyen d'un palier (31), et le moignon d'arbre (25) comprend un élément de couplage (28) qui s'engage avec un peu de jeu en coopération de formes dans le tronçon de centrage.

2. Agitateur selon la revendication 1,
**caractérisé en ce que** les pales d'agitateur (14) sont susceptibles d'être réglées avec des inclinaisons différentes par rapport à la verticale.

3. Agitateur selon la revendication 1 ou 2,
**caractérisé en ce que** le palier de centrage (16) comprend un tronçon de centrage (20) qui possède en vue de dessus la forme d'un polyèdre, en particulier d'un carré.

4. Agitateur selon l'une des revendications 1 à 3,
**caractérisé en ce que** le palier de centrage (16) comporte au niveau de la zone inférieure une ouverture hors de laquelle un matériau peut être refoulé lors de l'introduction de l'arbre d'agitateur (12) dans le palier de centrage (16).

5. Agitateur selon l'une des revendications 1 à 4,
**caractérisé en ce qu'**un appui (23) est agencé à l'intérieur du palier de centrage (16), ledit appui (23) étant doté d'une pointe de centrage (24) dirigée vers le haut.

6. Agitateur selon l'une des revendications 1 à 5,
**caractérisé en ce que** l'arbre d'agitateur (12) comprend un corps d'arbre (26) de forme tubulaire, dans lequel est monté le moignon d'arbre (25), ledit moignon d'arbre (25) dépassant une certaine distance le corps d'arbre (26), et un ou plusieurs paliers à billes, à roulements, à rouleaux et/ou à aiguilles (32) sont prévus entre le moignon d'arbre (25) et le corps d'arbre (26).

7. Agitateur selon l'une des revendications 1 à 6,
**caractérisé en ce que** les pales d'agitateur (14) sont susceptibles d'être fixées sur l'arbre d'agitateur (18) à des positions de l'arbre d'agitateur (12) qui peuvent être librement choisies.

8. Fermenteur, comprenant
- un boîtier (2) comprenant au moins une plaque de fond (4) et une ou plusieurs parois latérales (5) qui entourent la plaque de fond (4),
- un agitateur (1) qui est réalisé selon l'une des revendications 1 à 7,
- un système d'introduction (26) pour l'amenée de matériaux de départ, et
- une ouverture de prélèvement (41 ),
le système d'introduction (36) étant agencé pour l'amenée des matériaux de départ au niveau de la région supérieure du fermenteur (1), et l'ouverture de prélèvement (41) étant agencée au niveau de la région inférieure du fermenteur (1).

9. Fermenteur selon la revendication 8,
**caractérisé en ce que** l'ouverture de prélèvement (41) est agencée sur le fermenteur (1) diamétralement à l'opposé par rapport au système d'introduction (36), et
**en ce que** le fermenteur (1) comprend plusieurs agitateurs (11).

10. Procédé pour le fonctionnement d'un fermenteur, dans lequel on utilise un fermenteur selon la revendication 8 ou 9,
des matériaux de départ sont introduits au niveau de la région supérieure du fermenteur (1) pour une fermentation à sec, et le substrat fermenté est extrait au niveau de la région inférieure du fermenteur (1).

11. Procédé selon la revendication 10,
**caractérisé en ce que** les matériaux de départ présentent une teneur en matières sèches d'au moins 25 %.

12. Procédé selon l'une des revendications 10 ou 11,
**caractérisé en ce que** le substrat du fermenteur (1) est mélangé radialement avec l'agitateur (11), de sorte qu'il s'établit plusieurs zones de dégradation (43, 44, 45) en couches les unes au-dessus des autres.

13. Procédé selon l'une des revendications 10 à 12,
**caractérisé en ce que** l'inclinaison des pales d'agitateur (14) par rapport à la verticale est dans la plage de 20° à 70° et de préférence dans la plage de 30° à 60°.

14. Procédé selon l'une des revendications 10 à 13,
**caractérisé en ce que** l'arbre d'agitateur (12) qui se trouve dans le fermenteur est mis en fonctionnement avec une vitesse de rotation maximum de 60 tr/m.

15. Procédé pour le fonctionnement d'un fermenteur, dans lequel le fermenteur comprend un boîtier (2) avec un couvercle (6) et un agitateur (11) selon l'une des revendications 1 à 7, avec un arbre d'agitateur vertical et plusieurs pales d'agitateur (14) dépassant horizontalement sur l'arbre d'agitateur, et une ouverture (10) est ménagée dans le couvercle (6), dans lequel
lors de l'introduction et de l'extraction de l'agitateur (11) par rotation de l'arbre d'agitateur, les pales d'agitateur (14) sont orientées individuellement vis-à-vis de l'ouverture (10), de sorte que l'agitateur peut être introduit et extrait à travers une ouverture (10) qui est légèrement plus grande que la longueur des pales d'agitateur individuelles (14).
